# FASCICULE DE BREVET EUROPEEN

(11) **EP 1 072 251 B1**
(45) Date de publication et mention de la délivrance du brevet: **09.04.2003**
(21) Numéro de dépôt: 00113911.2
(22) Date de dépôt: 30.06.2000
(51) Int. Cl.: A61K 7/06, A61K 7/48

(54) **Préparations cosmetiques contenant des esters de N-acylaminoacides**
Kosmetische Zusammensetzungen, die N-Acyl-Aminosäureestern enthalten
Cosmetic compositions containing N-acyl-aminoacid esters

(30) Priorité: 21.07.1999 FR 9909574
(43) Date de publication de la demande: 31.01.2001
(73) Titulaire: LABORATOIRES ASEPTA SAM, 98000 Monaco (MC)
(72) Inventeur: Dencausse, Laurent, c/o OFFICE MED. DE BREVETS, 24 Rue Massena, 06000 Nice (FR); Clamou, Jean Luc, c/o OFFICE MED. DE BREVET, 24 Rue Massena, 06000 Nice (FR); Lacroix, Georges, c/o OFFICE MED. DE BREVET, 24 Rue Massena, 06000 Nice (FR)
(74) Mandataire: Hautier, Jean-Louis

(56) Documents cités:
- EP-A- 0 839 515
- EP-A- 0 882 702
- EP-A- 0 928 608
- DE-A- 2 260 640
- DE-A- 19 749 556
- FR-A- 2 074 498
- FR-A- 2 203 806

## Description

L'invention conceme la préparation de nouveaux esters de N-acylaminoacides. Les dérivés sont obtenus par N-acylation ou mieux par N-polyacylation d'acides aminés issus de protéines naturelles préalablement enrichis par un ou plusieurs acides aminés spécifiques. Les dits acides aminés spécifiques sont sélectionnés en fonction de leurs propriétés intrinsèques.

L'invention trouve une application préférentielle dans des compositions cosmétiques, dermatologiques et pharmaceutiques.

Actuellement, les N-acylaminoacides qui sont couramment employés dans l'industrie cosmétique concernent des N-acylaminoacides pris sous leur forme acide ou salifiée. L'utilisation d'esters de N-acylaminoacides est à ce jour peu décrite dans la littérature.

*Seule la demande de brevet EP 0839 515 A2 fait référence à l'utilisation de produits particuliers de ce type en cosmétique. Cependant, la demande de brevet EP 0839 515 A2 décrit exclusivement des esters de N-acylaminoacides ayant des groupes acylants à nombre impair d'atomes de carbone (plus spécialement C13 à C17) et des* acides *aminés pris individuellement.*

*EP 0 882 702: la présente invention concerne un perfectionnement au procédé de préparation de N-acylaminoacides ou de N-acylaminoacides esters à partir de* *protéines qui sont hydrolysées pour donner des acides aminés. Les réactions sont les suivantes :*
*1) l'estérification de chaque acide aminé en présence d'alcool,*
*2) l'acylation de l'ester en présence d'un halogénure d'acide, et éventuellement*
*3) la saponification du N-acylaminoacide ester en présence d'une base pour donner le N-acylaminoacide.*
La composition réalisée n'est pas réalisée à partir d'hydrolysat de protéine préalablement enrichie par un ou plusieurs acides aminés.

*EP 0 928 608*: de même la composition décrite dans ce brevet ne comprend pas d'hydrolysats de protéines.

*FR 2 074 498: la composition détergente est caractérisée en ce qu'elle contient, outre un détergent, un aminoacide portant un groupe acyle de 6 à 20 atomes de carbone sur l'atome d'azote, un sel ou un ester d'un tel aminoacide, et une enzyme. La compostion contient une enzyme et un aminoacide portant un groupe acyle de 6 à 20 atomes de carbones sur l'atome d'azote, un sel ou un ester d'un tel aminoacide, comme matière active détergente prédominante.*

Cette composition ne comporte pas de N-acylaminoacides.

L'objet de l'invention consiste à réaliser de nouveaux esters de N-acylaminoacides à partir d'un agent acylant ou d'un mélange renfermant plusieurs agents acylants et d'hydrolysats de protéines naturelles végétales ou animales préalablement enrichies par un ou plusieurs acides aminés pris individuellement conduisant ainsi à des hydrolysats de protéines enrichies. A notre connaissance, la réaction de condensation d'un agent acylant ou d'un mélange renfermant plusieurs agents acylants sur des hydrolysats de protéines enrichies n'a jamais été décrite.

L'association d'un agent acylant ou d'un mélange d'agents acylants avec un hydrolysat de protéines enrichies par un ou plusieurs acides aminés constitue donc une innovation. Les mélanges acylants pourront être binaires, temaires, quatemaires ou plus selon le type de N-acylaminoacides recherchés. Des compositions acylantes très différentes pourront être réalisées en faisant varier selon les besoins les proportions des différents halogénures d'acides constitutifs.

Les mélanges acylants destinés à être condensés sur les acides aminés ont pour formule générale I :

**(RCOX + R**_{**1**}**COX + ............ + R**_{**n**}**COX)**_{**m**}

X est un halogénure, R, R₁, ..., Rₙ sont des radicaux alkyles ou aryles saturés ou non et m est ≥ à 1.

Le nouveau procédé de préparation des N-acylaminoacides et de leurs esters (EP 0.882.702.A1) a été appliqué à un mélange d'halogénures d'acides. Les résultats montrent que la réactivité des différents halogénures d'acides vis à vis des acides aminés diffère peu. Ainsi, les dérivés N-polyacylés sont à la fois à l'image des acides aminés présents initialement mais également à l'image du mélange acylant réalisé. À titre d'exemple les mélanges acylants suivants ont été développés :
- Mélange de chlorure de palmitoyle/chlorure d'oléoyle 85/15 (mol/mol)
- Mélange de chlorure de lauroyle/chlorure d'undécénoyle 60/40 (mol/mol)
- Mélange de chlorure de lauroyle/chlorure de pentadécanoyle 99/1 (mol/mol)

La réalisation de tels mélanges permettra de cumuler les propriétés propres à chaques chaînes acylantes.
■ Les agents acylants classiquement utilisés dans la synthèse des esters du N-acylaminoacides sont :
   ■ l'acide caprylique pour ses propriétés bactériostatiques et fongistatiques,
   ■ l'acide undécylémique pour ses propriétés fongistatiques et de piège à odeurs,
   ■ l'acide laurique pour ses propriétés tensioactives,
   ■ l'acide palmitique pour ses qualités émollientes et sa prédominance dans l'organisme,
   ■ l'acide oléique et l'acide linoléique qui sont des acides gras essentiels doués de propriétés restructurantes au niveau de l'épiderme.

L'association d'acides aminés provenant d'hydrolysats de protéines naturelles et d'un ou de plusieurs acides aminés spécifiques est réalisée sur matière sèche. Les proportions de chaque acide aminé additionné pourront varier de 0,5 % à 70% et plus précisément de 5 à 60% en masse par rapport à l'hydrolysat.

Les différents hydrolysats de protéines sont choisis selon leur origine et suivant la nature des acides aminés qui les constituent. Ainsi, selon les esters de N-acylaminoacides recherchés, un hydrolysat de protéine bien particulier sera sélectionné. Les hydrolysats de protéines végétales (avoine, blé, soja) permettront d'obtenir naturellement des esters de N-acylaminoacides avec des proportions élevées en acides aminés diacides (acide glutamique, acide aspartique) alors que les hydrolysats de protéines de soie (fibroïne et séricine) conduiront à l'obtention d'esters de N-acylaminoacides riches en acides β-hydroxylés (sérine et thréonine).

Les différents acides aminés individuels destinés à enrichir et à optimiser les hydrolysats de protéines sont sélectionnés en fonction de leurs propriétés intrinsèques.

Ainsi, les acides aminés β-hydroxylés tels que la sérine, la thréonine ou la tyrosine seront choisis pour leurs propriétés hydratantes et leurs aptitudes à réaliser des interactions hydrogènes avec l'eau.

Des acides aminés essentiels pourront également être associés aux hydrolysats protéiques. La leucine sera par exemple retenue en raison de sa contribution à la cicatrisation de la peau alors que la méthionine interviendra de manière à constituer un apport exogène en souffre particulièrement recherché dans le traitement et dans le soin des phanères (cils, ongles, cheveux).

À titre d'exemple, les hydrolysats de protéines enrichis par les acides aminés suivants ont été développés :
- Acides aminés de soie additionnés de 20 à 60% en masse de sérine ;
- Acides aminés de soie additionnés de 5 à 10% en masse de méthionine ;
   Acides aminés d'avoine additionnés de 20 à 60% en masse de leucine ;
- Acides aminés de blé additionnés de 10 à 30% en masse de thréonine.

Selon l'objet inventif de ce brevet, différents esters de N-acylaminoacides pourront être composés selon :
■ la nature de l'agent acylant ou des mélanges acylants utilisés ;
■ l'origine des hydrolysats protéiques utilisés ;
■ la nature du ou des acides aminés utilisés pour enrichir les différents hydrolysats de protéines.

La composition cosmétique est du type comprenant des mélanges de N-acylaminoacides pris sous leur forme d'esters de N-acylaminoacides, les mélanges acides aminés proviennent d'hydrolyse de protéines (végétales ou animales) pour former ainsi des mélanges d'acides aminés, du type comprenant des esters N-acylaminoacides à partir d'un mélange de plusieurs agents acylants, caractérisée par le fait que les mélanges acylants peuvent être binaires, ternaires, quaternaires ou plus selon le type de N-acylaminoacides recherché, qu'il comprend une combinaison :
- d'acides aminés issus de protéines naturelles préalablement enrichis artificiellement par un ou plusieurs acides aminés pris individuellement.

Les proportions de chaque acide aminé additionné peuvent varier de 0,5 à 70 % en masse par rapport à l'hydrolysat.

Les proportions de chaque acide aminé additionné peuvent varier de 5 à 60 % en masse par rapport à l'hydrolysat.

Les mélanges acylants sont les suivants :
- mélange de chlorure de palmitoyle/chlorure d'oléoyle 85/15 (mol/mol).

Les mélanges acylants sont les suivants :
- mélange de chlorure de lauroyle/chlorure d'undécénoyle 60/40 (mol/mol).

Les mélanges acylants sont les suivants :
- mélange de chlorure de lauroyle/chlorure de pentadécanoyle 99/1 (mol/mol).

L'hydrolysat de protéines enrichis par les acides aminés est le suivant :
- acides aminés de soie additionnés de 20 à 60 % en masse de sérine.

L'hydrolysat de protéines enrichis par les acides aminés est le suivant :
- acides aminés de soie additionnés de 5 à 10 % en masse de méthionine.

L'hydrolysat de protéines enrichis par les acides aminés est le suivant :
- acides aminés de soie additionnés de 20 à 60 % en masse de leucine.

L'hydrolysat de protéines enrichis par les acides aminés est le suivant :
- acides aminés de soie additionnés de 10 à 30 % en masse de thréonine.

Le tableau 1 permet de décrire sommairement les différentes associations permettant de réaliser de nouveaux esters de N-acylaminoacides.

**Tableau 1 :**

| Associations permettant de réaliser de nouveaux esters de N-acylaminoacides | | |
|---|---|---|
| **Sous forme d'esters** | **1 seul agent acylant utilisé** | **Mélange de plusieurs agents acylants** |
| **Hydrolysat de protéine** | - | ⊗ |
| **Hydrolysat de protéine enrichi par 1 ou plusieurs acides aminés individuels** | + | + |
| **1 acide aminé pris individuellement** | - | ⊗ |
| +: **Associations pouvant être à l'origine de nouveaux esters de N-acylaminoacides.** | | |
| **-: Esters de N-acylaminoacides déjà décrits antérieurement.** | | |
| **⊗ : Esters de N-acylaminoacides qui sortent du cadre de développement prévu.** | | |

Selon les associations décrites dans le tableau 1, les nouveaux esters de N-acylaminoacides suivants ont été développés :

### 1 seul agent acylant associe à 1 hydrolysat de protéines enrichi par 1 acide aminé spécifique :

□ N-Lauroyl - Soie/Sérine (80/20) méthyl ester ;
□ N-Undécénoyl - Soie/Sérine (70/30) méthyl ester ;
□ N-Palmitoyl - Soie/Sérine (40/60) méthyl ester ;
□ N-Palmitoyl - Blé/Thréonine (70/30) méthyl ester.

### Mélange d'agents acylante associé à 1 hydrolysat de protéines enrichi par 1 acide aminé spécifique :

□ N-Palmitoyl/Oléoyl (85/15) - Soie/Sérine (40/60) méthyl ester ;
□ N-PalmitoyI/Oléoyl (90/10) - Soie/Méthionine (90/10) méthyl ester ;
□ N-Palmitoyl/Oléoyl (85/15) - Avoine/Leucine (40/60) méthyl ester ;
□ N-Lauroyl/Pentadécanoyl (99/1) - Soie/Sérine (70/30) méthyl ester.

Ces différents N-acylaminoacides esters de protéines naturelles additionnées trouvent une application préférentielle en podologie (compositions destinées à la prévention et au soin des mycoses), en cosmétologie pour l'entretien des phanères et des capillaires (préparations fortifiantes et traitantes) ainsi que pour les soins cosmétiques du visage et des mains (formulations destinées à favoriser l'hydratation cutanée). Les nouveaux esters de N-acylaminoacides de la présente invention peuvent être incorporés dans de nombreuses formulations émulsionnées (crèmes, gels, shampooings), des solutions (lotions, sprays) et des préparations pulvérulentes-(poudres, atomisats).

Dans les produits dermo-cosmétiques de soin du visage et des mains les esters de N-acylaminoacides présentent un pouvoir substantif à de faibles concentrations et une pénétration significative de la barrière cutanée améliorant ainsi les propriétés mécaniques et le taux d'hydratation de la peau. Dans ce domaine, les esters N-Palmitoyl - Soie/Sérine (40/60) sont particulièrement actifs.

A ce titre, l'effet hydratant d'une émulsion huile/eau renfermant 0,5% d'esters méthyliques de N-palmitoyl soie/sérine (40/60,m/m) a été démontré sur dix volontaires sains de sexe féminin (âge moyen 37±4ans) présentant une peau sèche au niveau des jambes (étude n°99363 réalisée sous contrôle dermatologique).

L'application standardisée et isolée de l'échantillon sur une jambe de chacune des dix volontaires (l'autre jambe servant de témoin) a permis de contrôler les variations en pourcentage (Δ)%) du taux d'hydratation cutané à t 1 heure, t 3 heures et t 6 heures. Les résultats sont rassemblés dans le graphique 1.

L'augmentation du taux d'hydratation cutanée obtenu une heure (64±8%), trois heures (52±7%), et six heures (40±7%) après l'application de la crème confirme l'excellent pouvoir hydratant du produit in vivo sur l'homme. Les variations obtenues sont statistiquement significatives (p<0,001 - test de Student bilatéral sur séries appariées).

Dans les préparations capillaires une absorption et une pénétration caractéristique des esters de N-acylaminoacides a été observée sur la cuticule des cheveux notamment lors de l'utilisation du N-Undécénoyl - Soie/Sérine (70/30) méthyl ester qui en raison de son faible poids moléculaire présente une substantivité intéressante. L'association du chlorure d'undécénoyle comme agent acylant permet d'obtenir des esters de N-acylaminoacides qui présentent des propriétés fongicides très intéressantes dans le traitement des états pelliculaires des cheveux et dans le cadre du soin des mycoses. D'autre part, les esters de N-acylaminoacides présentent une solubilité importante dans les solvants alcooliques ce qui autorise la préparation de lotions, d'ampoules capillaires antipelliculaires et de solutions antimycosiques spécifiques.

Les exemples qui suivent décrivent en détail la préparation de certains N-acylaminoacides esters de protéines naturelles additionnées et leurs applications cosmétiques.

### Exemple 1

### Synthèse du N-Palmitoyl-Soie/Sérine (40/60) méthyl ester.

### (1) Préparation du substrat protéique de soie enrichi en sérine

Dans un erlenmeyer, 10g d'acides aminés de soie atomisés sont additionnés de 15g de sérine selon les proportions massiques correspondants au mélange binaire 40% d'acides aminés de soie atomisée et 60% de sérine.

La composition en acides aminés de la protéine de soie qui est utilisée est donnée dans le tableau 2.

**Tableau 2**

| Aminogramme des acides aminés atomisés de la soie (% m/m) | | | |
|---|---|---|---|
| **Acides aminés** | **% massiques** | **Acides aminés** | **% massiques** |
| Glycine | 45,24 | Tyrosine | 0,14 |
| Alanine | 42,90 | Isoleucine | 0,05 |
| Sérine | 10,29 | Méthionine | 0,04 |
| A. Glutamique | 0,46 | Phénylalanine | 0,03 |
| Valine | 0,44 | Leucine | 0,03 |
| A. Aspartique | 0,36 | Cystéine | 0,02 |

Le pourcentage initial de sérine dans la soie utilisée est de 10,29%. L'addition de 15g de sérine permet d'augmenter artificiellement les proportions globales de sérine pour obtenir un pourcentage total égal à 64,12%. Cette addition de sérine permet de disposer d'un hydrolysat de soie particulièrement riche en β-hydroxyacides.

### (2) Estérification de l'hydrolysat de soie enrichi en sérine

L'estérification des 25g d'hydrolysat de soie enrichi en sérine est réalisée dans un ballon bicol de 250ml. Les équivalences de réactifs à additionner sont calculées suivant le nombre total de moles d'acides aminés à estérifier. Le tableau 3 présente les proportions molaires d'acides aminés dans 25g de mélange soie/serine (40/60 ; m/m).

**Tableau 3**

| Proportions molaires des différents acides aminés dans 25g de mélange soie/sérine (40/60;m/m) | | | |
|---|---|---|---|
| **Acides aminés** | **Proportions molaires** | **Acides aminés** | **Proportions molaires** |
| Glycine | 0,0602 | Tyrosine | 7,72.10⁻⁵ |
| Alanine | 0,0481 | Isoleucine | 3,81.10⁻⁵ |
| Sérine (totale) | 9,8.10⁻³ + 0,142 | Méthionine | 2.68.10⁻⁵ |
| A. Glutamique | 3,12.10⁻⁴ | Phénylalanine | 1,81.10⁻⁵ |
| Valine | 3,75.10⁻⁴ | Leucine | 1,81.10⁻⁵ |
| A. Aspartique | 2,70.10⁻⁴ | Cystéine | 1,65.10⁻⁵ |

Dans 25g de mélange soie/sérine (40/60 ; m/m), le nombre total de moles estérifiées est égal à 0,262. Le mélange d'acides aminés est additionné de 150 ml de méthanol puis de 0,7eq de SOCl₂ soit 0,178 mole, soit une masse de 21,20g et un volume de 13,0ml. Le chlorure de thionyle est additionné goutte à goutte tout en maintenant le mélange méthanolique d'acides aminés à une température proche de 5°C (bain de glace). Lorsque la totalité du SOCl₂ a été additionnée, le mélange réactionnel est ensuite chauffé à reflux pendant 1 heure. Le méthanol et le SOCl₂ en excès sont éliminés à l'évaporateur rotatif sous vide. Les esters d'acides aminés sous forme de chlorhydrate sont de couleur blanche. La masse obtenue est de 36,5g ce qui correspond à un rendement d'estérification par rapport aux acides aminés de 96%. Le nombre total de moles d'acides aminés à acyler est alors de 0,251 mole.

### (3) Synthèse des N-palmitoyl - Soie/Serine (40/60) méthyl ester

Les 36,5g d'esters d'acides aminés sous forme de chlorhydrate sont placés dans un ballon bicol de 1 litre en présence de 400ml de chlorure de méthylène et de 59,5ml de triéthylamine (1,7 équivalents/acides aminés estérifiés). Le mélange est refroidi entre 0-5°C puis additionné de 0,98eq soit 0,246 mole ou 67,6g de chlorure de palmitoyle en solution dans 30ml de chlorure de méthylène. Après addition de l'agent acylant, le mélange est agité pendant une heure puis transféré dans une ampoule à décanter. La phase organique est lavée 4 fois par 250ml d'eau acidulée pour éliminer la triéthylamine en excès. La phase organique est séchée, filtrée puis évaporée sous vide.

Les esters méthyliques de N-palmitoyl - Soie/Sérine (40/60) sont purifiés par recristallisation dans 200ml d'hexane puis séchés à l'étuve à 40°C.

77,5g de N-palmitoyl - Soie/Sérine (40/60) méthyl ester sont obtenus sous forme de poudre blanche avec une odeur caractéristique.

Le rendement massique de la synthèse est de 85,7%.

Les propriétés physico-chimiques des esters méthyliques des N-palmitoyl soie/serine (40/60;m/m) sont donnés ci-dessous :
**Nombre d'ondes IR caractéristiques (en cm**^{**-1**}**)**
719, 1227, 1544, 1645, 1727, 1742, 2850, 2919, 3314, 3497.

| **Analyse élémentaire** | | | | |
|---|---|---|---|---|
| Valeur calculée | C : 68,26 | H : 11,19 | N : 4,03 | O : 16,52 |
| Valeur trouvée | C : 69,58 | H : 11,07 | N : 3,16 | O : 16,19 |

### Point de fusion

74°C (± 2°C)

### Exemple 2

### Synthèse du N-Undécénoyl - Soie/Sérine (70/30) méthyl ester.

### (1) Préparation du substrat protéique de soie enrichi en sérine

Dans un erlenmeyer, 10g d'acides aminés de soie atomisés sont additionnés de 4,3g de sérine selon les proportions massiques correspondants au mélange binaire 70% d'acides aminés de soie atomisée et 30% de sérine. La composition en acides aminés de la protéine de soie qui est utilisée est celle donnée dans le tableau 2 de l'exemple 1. L'addition de 4,3g de sérine permet d'augmenter artificiellement les proportions globales de sérine et d'obtenir un pourcentage total en sérine égal à 37%. Cette addition de sérine permet de disposer d'un hydrolysat de soie particulièrement riche en β-hydroxyacides.

### (2) Estérification de l'hydrolysat de soie enrichi en sérine

L'estérification des 14,3g d'hydrolysat de soie enrichi en sérine est réalisée dans un ballon bicol de 250ml. Les équivalences de réactifs à additionner sont calculées suivant le nombre total de moles d'acides aminés à estérifier. Le tableau 4 présente les proportions molaires des différents acides aminés dans 14,3g de mélange soie/sérine (70/30;m/m)

**Tableau 4**

| Proportions molaires des différents acides aminés dans 14,3g de mélange soie/sérine (70/30;m/m) | | | |
|---|---|---|---|
| **Acides aminés** | **Proportions molaires** | **Acides aminés** | **Proportions molaires** |
| Glycine | 0,0602 | Tyrosine | 7,72.10⁻⁵ |
| Alanine | 0,0481 | Isoleucine | 3,81.10⁻⁵ |
| Sérine (totale) | 9,8.10⁻³ + 0,04 | Méthionine | 2,68.10⁻⁵ |
| A. Glutamique | 3,12.10⁻⁴ | Phénylalanine | 1,81.10⁻⁵ |
| Valine | 3,75.10⁻⁴ | Leucine | 1,81.10⁻⁵ |
| A. Aspartique | 2,70.10⁻⁴ | Cystéine | 1,65.10⁻⁵ |

Dans 14,3 g de mélange soie/sérine (70/30 ; m/m) le nombre total de moles estérifiées est égal à 0,159. Le mélange d'acides aminés est additionné de 150 ml de méthanol puis de 0,7eq de SOCl₂ soit 0,11 mole, soit une masse de 13,25g et un volume de 8,1ml. Le chlorure de thionyle est additionné goutte à goutte tout en maintenant le mélange méthanolique d'acides aminés à une température proche de 5°C (bain de glace). Lorsque la totalité du SOCl₂ a été additionnée, le mélange réactionnel est ensuite chauffé à reflux pendant 1 heure. Le méthanol et le SOCl₂ en excès sont éliminés à l'évaporateur rotatif sous vide. Les esters d'acides aminés sous forme de chlorhydrate sont de couleur blanche. La masse obtenue est de 21,4g ce qui correspond à un rendement d'estérification par rapport aux acides aminés de 96%.

Le nombre total de mole d'acides aminés à acyler est alors de 0,152 mole.

### (3) Synthèse des N-Undécénoyl - Soie/Sérine (70/30) méthyl ester

Les 21,4g d'esters d'acides aminés sous forme de chlorhydrate sont placés dans un ballon bicol de 1 litre en présence de 400ml de chlorure de méthylène et de 36,0ml de triéthylamine (1,7équivalents/acides aminés estérifiés). Le mélange est refroidi entre 0-5°C puis additionné de 30,2g (0,98 équivalent/acides aminés estérifiés) de chlorure d'undécénoyle en solution dans 30ml de chlorure de méthylène. Après addition de l'agent acylant, le mélange est agité pendant une heure puis transféré dans une ampoule à décanter. La phase organique est lavée 4 fois par 250ml d'eau acidulée pour éliminer la triéthylamine en excès. La phase organique est séchée, filtrée puis évaporée sous vide.

Les esters méthyliques de N-Undécénoyl - Soie/Sérine (70/30) sont purifiés par recristallisation dans 200ml d'hexane puis séchés à l'étuve ventilée à 30°C.

31g de N-Undécénoyl - Soie/Sérine (70/30) méthyl ester sont obtenus sous forme de poudre blanche avec une odeur caractéristique de l'acide undécylénique.

Le rendement massique de la synthèse est de 72%.

Les propriétés physico-chimiques des esters méthyliques du N-Undécénoyl soie/sérine (70/30;m/m) sont donnés ci-dessous :
**Nombre d'ondes IR caractéristiques (en cm**^{**-1**}**)**
910, 1210, 1544, 1645, 1728, 1748, 2852, 2925, 3305, 3497

| **Analyse élémentaire** | | | | |
|---|---|---|---|---|
| Valeur calculée | C : 65,26 | H : 9,82 | N : 5,19 | O : 19,73 |
| Valeur trouvée | C : 65,52 | H : 9,57 | N : 4,46 | O : 20,45 |

### Point de fusion

<40°C (± 2°C)

### Exemple 3

### Applications des N-acylaminoacides esters de protéines naturelles additionnées à des produits cosmétiques destinés à l'hygiène corporelle et aux soins cosmétiques.

| ***• Composition crème hydratante pour les pieds*** | |
|---|---|
| **INGRÉDIENTS** | **%** |
| ISOPROPYL PALMITATE | 15 |
| GLYCERYL STEARATE | 5 |
| CETEARETH-20 | 3 |
| LANOLIN | 2 |
| GLYCERIN | 10 |
| N-PALMITOYL/-SOIE/SÉRINE MÉTHYL ESTER (40160) | 3 |
| ALPHA TOCOPHÉROL | 0,5 |
| PARFUM (FRAGRANCE) | Quantité nécessaire (proper amount) |
| CONSERVATEURS (Antiseptics) | Quantité nécessaire (proper amount) |
| AQUA (Purified water) | qsp 100 (balance) |

| ***• Composition crème antitranspirante pieds pour la prévention des mycoses*** | |
|---|---|
| **INGRÉDIENTS** | **%** |
| GLYCÉRYL STÉARATE | 7 |
| GLYCÉRINE | 5 |
| CETEARETH-12 | 4 |
| PARAFFINUM LIQUIDUM | 3 |
| ALUMINIUM CHLORHYDRATE | 10 |
| UNOÉCÉNOYL - SOIE/SERINE MÉTHYL ESTER 70/30 | 3 |
| ALPHA TOCOPHÉROL | 0,2% |
| FRAGRANCE | Quantité nécessaire (proper amount) |
| CONSERVATEURS (Antiseptics) | Quantité nécessaire (proper amount) |
| AQUA (Purified water) | qsp 100 (balance) |

| ***• Composition savon crème purifiant peaux grasses et acnéiques*** | |
|---|---|
| **INGREDIENTS** | **%** |
| GLYCÉRYL STÉARATE | 8 |
| PARAFFINUM LIQUIDUM | 4 |
| SODIUM TEA HYDROLIZED PROTEIN | 3 |
| UNDÉCÉNOYL - SOIE/SERINE MÉTHYL ESTER 70/30) | 3 |
| STEARIC ACID | Quantité nécessaire (proper amount) |
| CONSERVATEURS (Antiseptics) | Quantité nécessaire (proper amount) |
| FRAGRANCE | Quantité nécessaire (proper amount) |
| AQUA (Purified water) | Qsp 100 (balance) |

| **• *Composition shampooing fortifiant*** | |
|---|---|
| **INGRÉDIENTS** | **%** |
| SODIUM COCETH SULFATE | 10 |
| SODIUM LAURYL SULFATE | 15 |
| COCOAMIDOPROPYL BETAÏNE | 7 |
| PEG 40 GLYCERYL COCOATE | 4 |
| COCAMIDE DEA | 3 |
| N-LAUROYL/PENTADÉCANOYL (99/1)-SOIE/SÉRINE MÉTHYL ESTER (70/30) | 1 |
| FRAGRANCE | Quantité nécessaire (proper amount) |
| | Quantité nécessaire (proper amount) |
| CONSERVATEURS (Antiseptics) | Quantité nécessaire (proper amount) |
| AQUA (PURIFIED WATER) | |

| ***• Composition lotion antipelliculaire*** | |
|---|---|
| **INGREDIENTS** | **%** |
| ALCOOL DÉNATURÉ | 45 |
| UNDÉCÉNOYL - SOIE/SERINE MÉTHYL ESTER (70/30) | 2 |
| ROSMARINUS OFFICINALIS | 0,2 |
| THYMUS VULGARIS | 1 |
| AQUA (PURIFIED WATER) | qsp 100 (balance) |

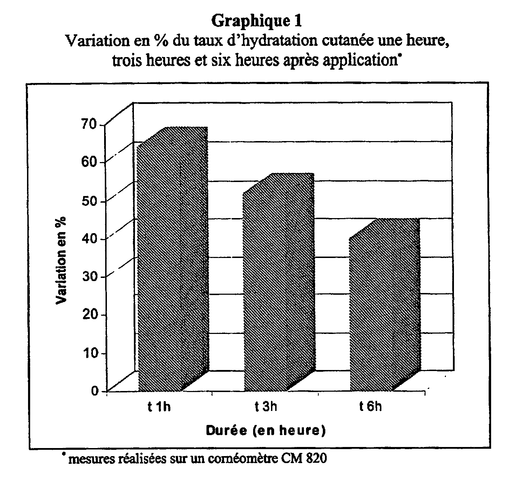

## Revendications

1. Composition cosmétique comprenant des mélanges de N-acylaminoacides pris sous leur forme d'esters de N-acylaminoacides, les acides aminés proviennent d'hydrolyse de protéines (végétales ou animales) pour former ainsi des mélanges d'acides aminés, comprenant des esters N-acylaminoacides à partir d'un agent acylant, **caractérisée par le fait**
- **que** le ou les agents acylants sont des halogénures d'acides pouvant être binaires, temaires, quaternaires, ou plus suivant le type de N-acylaminoacides recherchés.
- **Que** les acides aminés utilisés pour la synthèse des esters de N-acylaminoacides sont issus de protéines naturelles végétales ou animales préalablement enrichis artificiellement par un ou plusieurs acides aminés pris individuellement conduisant ainsi à des hydrolysats de protéines enrichis,
- **Que** par réaction de condensation acylante les dits « hydrolysats de protéines enrichis » sont associés selon les cas à un ou plusieurs halogénures d'acides pour conduire à des mélanges d'esters de N-acylaminoacides.

2. Composition selon la revendication 1 **caractérisée par le fait,**
**que** les proportions de chaque acide aminé additionné peuvent varier de 0.5 % à 70 % en masse par rapport à l'hydrolysat.

3. Composition selon la revendication 1 **caractérisée par le fait**
**que** les proportions de chaque acide aminé additionné peuvent varier de 5 à 60 % en masse par rapport à l'hydrolysat.

4. Composition selon la revendication 1 **caractérisée par le fait,**
**que** l'halogénure d'acide utilisé comme agent acylant est le chlorure de capryloyle.

5. Composition selon la revendication 1 **caractérisée par le fait que**,
que l'halogénure d'acide utilisé comme agent acylant est le chlorure d'undécénoyle.

6. Composition selon la revendication 1 **caractérisée par le fait que**,
que l'halogénure d'acide utilisé comme agent acylant est le chlorure de palmitoyle.

7. Composition selon la revendication 1 **caractérisée par le fait**
**que** les mélanges acylants sont les suivants :
- mélange de chlorure de palmitoyle/chlorure d'oléoyle 85/15 (mol/mol).

8. Composition selon la revendication 1 **caractérisée par le fait**
**que** les mélanges acylants sont les suivants :
mélanges de chlorure de lauroyle/chlorure d'undécénoyle 60/40 (mol/mol).

9. Composition selon la revendication 1 **caractérisée par le fait**
**que** les mélanges acylants sont les suivants :
mélanges de chlorure de lauroyle/chlorure de pentadécanoyle 99/1 (mol/mol).

10. Composition selon la revendication 1 **caractérisée par le fait**
**que** l'hydrolysat de protéines enrichi par les acides aminés est le suivant :
- acides aminés de soie additionnés de 20 à 60 % en masse de sérine.

11. Composition selon la revendication 1 **caractérisée par le fait**
**que** l'hydrolysat de protéines enrichi par les acides aminés est le suivant :
- acides aminés de soie additionnés de 5 à 10 % en masse de méthionine.

12. Composition selon la revendication 1 **caractérisée par le fait**
**que** l'hydrolysat de protéines enrichi par les acides aminés est le suivant :
- acides aminés de soie additionnés de 20 à 60 % en masse de leucine.

13. Composition selon la revendication 1 **caractérisée par le fait**
**que** l'hydrolysat de protéines enrichi par les acides aminés est le suivant :
- acides aminés de soie additionnés de 10 à 30 % en masse de thréonine.

14. Utilisation d'une composition selon l'une quelconque des revendications 1 à 13 **caractérisée par le fait**
**que** dans les produits dermo-cosmétiques de soin du visage et des mains, les esters de N-acylaminoacides présentent un pouvoir substantif à de faibles concentrations et une pénétration significative de la barrière cutanée améliorant ainsi les propriétés mécaniques et le taux d'hydratation de la peau.

15. Utilisation selon la revendication 14 **caractérisée par le fait**
**que** dans ce domaine les esters M-Palmitoyle - Soie/sérine (40/60) sont particulièrement actifs.

16. Produit cosmétique et/ou dermatologique composé selon l'une quelconque des revendications 1 à 13 pour une application destinée à l'hygiène corporelle et aux soins cosmétiques.

## Claims

1. Cosmetic composition comprising mixtures of N-acylaminoacids taken in their N-acylaminoacid ester form, the amino acids result from protein hydrolysis (plant or animal) so as to form mixtures of amino acids, including N-acylaminoacid esters from an acylant agent, **characterized in that**
- the acylant agents are acid halides that may be binary, ternary, quaternary, or more depending on the required type of N-acylaminoacids.
- the amino acids used for the synthesis of N-acylaminoacid esters are drawn from natural plant or animal proteins previously artificially enriched by one or more amino acids taken individually thereby leading to new enriched protein hydrolysates,
- by reaction of acylant condensation the said "enriched protein hydrolysates" are associated according to the case with one or more acid halides to produce N-acylaminoacid ester mixtures.

2. Composition according to claim 1, **characterized in that**,
the proportions of each added amino acid can vary from 0.5% to 70% by weight compared to the hydrolysate.

3. Composition according to claim 1, **characterized in that**
the proportions of each added amino acid can vary from 5 to 60% by weight compared to the hydrolysate.

4. Composition according to claim 1, **characterized in that**,
that the acid halide used as acylant agent is the capryloyl chloride.

5. Composition according to claim 1, **characterized in that**,
the acid halide used as acylant agent is the undecenoyl chloride.

6. Composition according to claim 1, **characterized in that**,
the acid halide used as acylant agent is chloride palmitoyl.

7. Composition according to claim 1, **characterized in that**
the acylant mixtures are as follows:
- mixture of 85/15 palmitoyl chloride/oleoyl chloride (mol/mol).

8. Composition according to claim 1, **characterized in that** the acylant mixtures are as follows:
- mixtures of 60/40 lauroyl chloride/undecenoyl chloride (mol/mol).

9. Composition according to claim 1, **characterized in that** the acylant mixtures are as follows:
- mixtures of 99/1 lauroyl chloride/undecenoyl chloride (mol/mol).

10. Composition according to claim 1, **characterized in that**
the amino acid enriched hydrolysate is as follows:
- silk amino acids with 20 to 60 % added serine by weight.

11. Composition according to claim 1, **characterized in that**
the amino acid enriched hydrolysate is as follows:
- silk amino acids with 5 to 10 % added methionine by weight.

12. Composition according to claim 1, **characterized in that**
the amino acid enriched hydrolysate is as follows:
- silk amino acids with 20 to 60 % added leucine by weight.

13. Composition according to claim 1, **characterized in that**
the amino acid enriched hydrolysate is as follows:
- silk amino acids with 10 to 30 % added threonine by weight.

14. Application of a composition according to any one of the claims 1 to 13 **characterized in that**
in dermo-cosmetic face and hand care products, the N-acylaminoacid esters present a substantive capacity at weak concentrations and a significant penetration of the cutaneous barrier thereby improving the mechanical properties and the skin hydration rate.

15. Composition according to claim 14, **characterized in that** in this field the esters M-Palmitoyl ― Silk/serine (40/60) are particularly active.

16. Cosmetic and/or dermatological product composed according to any one of the claims 1 to 13 for personal hygiene and cosmetic care applications.

## Patentansprüche

1. Kosmetische Zusammensetzung bestehend aus Mischungen von N-Acylaminosäuren in Form von Estem der N-Acylaminosäuren, in der die durch Hydrolyse tierischer oder pflanzlicher Proteinen gewonnenen Aminosäuren Mischungen bilden, in denen unter Mitwirkung eines Acylbildners N-Acylaminosäure-Ester vorhanden sind, **gekennzeichnet dadurch, daß**
- der oder die Acylbildner je nach Typ der gewünschten N-Acylaminosäuren binäre, ternäre, quaternäre oder mehrfache Säurehalogenide sein können,
- die zur Synthese der N-Acylaminosäure-Ester verwendeten Aminosäuren aus natürlichen pflanzlichen oder tierischen Proteinen stammen, die zuvor künstlich mit einer oder mehrerer, einzeln zugefügten Aminosäuren angereichert wurden, was zur Bildung von angereicherten Proteinhydolysaten führt,
- durch eine acylbildende Kondensation die genannten "angereicherten Proteinhydrolysate" je nach Fall mit einem oder mehreren Säurehalogeniden verbunden und so Mischungen von N-Acylaminosäure-Estern gebildet werden.

2. Zusammensetzung nach Anspruch 1, **gekennzeichnet dadurch, daß** die Mengenanteile jeder hinzugefügten Aminosäure zwischen 0,5 und 70 Gewichtsprozent des Hydrolysats liegen können.

3. Zusammensetzung gemäß Anspruch 1, **gekennzeichnet dadurch, daß** die Mengenanteile jeder hinzugefügten Aminosäure zwischen 5 und 60 Gewichtsprozent des Hydrolysats liegen können.

4. Zusammensetzung gemäß Anspruch 1, **gekennzeichnet dadurch, daß** das zur Acylierung verwendete Säurehalogenid Capryloylchlorid ist.

5. Zusammensetzung gemäß Anspruch 1, **gekennzeichnet dadurch, daß** das zur Acylierung verwendete Säurehalogenid Undecenoylchlorid ist.

6. Zusammensetzung gemäß Anspruch 1, **gekennzeichnet dadurch, daß** das zur Acylierung verwendetet Säurehalogenid Palmitoylchlorid ist.

7. Zusammensetzung gemäß Anspruch 1, **gekennzeichnet durch** folgende Acylierungsmischungen:
- Palmitoylchlorid und Oleoylchlorid im Verhältnis von 85 zu 15 (mol/mol)

8. Zusammensetzung gemäß Anspruch 1, **gekennzeichnet durch** folgende Acylierungsmischungen:
- Lauroylchlorid und Undecenoylchloid im Verhältnis von 60 zu 40 (mol/mol).

9. Zusammensetzung gemäß Anspruch 1, **gekennzeichnet durch** folgende Acylierungsmischungen:
- Lauroylchlorid und Pentadecanoylchlorid im Verhältnis von 99 zu 1 (mol/mol)

10. Zusammensetzung gemäß Anspruch 1, **gekennzeichnet durch** folgendes, mit Aminosäuren angereichertes Proteinhydrolysat:
- Seiden-Aminosäuren mit Zusatz von 20 bis 60 Gewichtsprozent Serin

11. Zusammensetzung gemäß Anspruch 1, **gekennzeichnet durch** folgendes, mit Aminosäuren angereichertes Proteinhydrolysat:
- Seiden-Aminosäuren mit Zusatz von 5 bis 10 Gewichtsprozent an Methionin

12. Zusammensetzung gemäß Anspruch 1, **gekennzeichnet durch** folgendes, mit Aminosäuren angereichertes Proteinhydrolysat:
- Seiden-Aminosäuren mit Zusatz von 20 bis 60 Gewichtsprozent Leucin

13. Zusammensetzung gemäß Anspruch 1, **gekennzeichnet durch** folgendes, mit Aminosäuren angereichertes Proteinhydrolysat
- Seiden-Aminosäuren mit Zusatz von 10 bis 30 Gewichtsprozent Threonin

14. Verwendung einer Zusammensetzung nach einem der Ansprüche 1 bis 13, **gekennzeichnet dadurch, daß** die Ester der N-Acylaminosäuren in den Hautpflegemittel für Gesicht und Hände in geringen Mengen eine gute Wirksamkeit und eine signifikante Penetration der Hautbarriere zeigen, was zu einer Verbesserung der mechanischen Eigenschaften und des Hydratationsgrades der Haut führt.

15. Zusammensetzung gemäß Anspruch 14, **gekennzeichnet dadurch, daß** die M-Palmitoyl-Ester - Seide/Serin (40/60) in diesem Bereich besonders aktiv sind.

16. Kosmetikprodukt und/oder dermatologisches Produkt mit einer der in den Ansprüchen 1 bis 13 genannten Zusammensetzungen zur Anwendung in der Körperhygiene und für kosmetische Zwecke.
